# EUROPÄISCHE PATENTSCHRIFT

(11) **EP 1 560 585 B1**
(45) Veröffentlichungstag und Bekanntmachung des Hinweises auf die Patenterteilung: **01.08.2012**
(21) Anmeldenummer: 03772256.8
(22) Anmeldetag: 24.10.2003
(51) Int. Cl.: A61K 31/485, A61K 31/4725, A61K 9/20

(54) **GEGEN MISSBRAUCH GESICHERTE DARREICHUNGSFORM**
DOSAGE FORM THAT IS SAFEGUARDED FROM ABUSE
FORME GALENIQUE PROTEGEE CONTRE UN USAGE DETOURNE

(30) Priorität: 25.10.2002 DE 10250088
(43) Veröffentlichungstag der Anmeldung: 10.08.2005
(73) Patentinhaber: Grünenthal GmbH, 52078 Aachen (DE)
(72) Erfinder: BARTHOLOMÄUS, Johannes, 52080 Aachen (DE); LANGNER, Klaus-Dieter, 52078 Aachen (DE)
(74) Vertreter: Kutzenberger, Helga
(86) Internationale Anmeldenummer: PCT/EP2003/011785
(87) Internationale Veröffentlichungsnummer: WO 2004/037260

(56) Entgegenhaltungen:
- WO-A-02/092059
- US-A- 4 457 933
- US-A- 5 149 538
- US-A- 5 866 164

## Beschreibung

Die vorliegende Erfindung betrifft eine gegen Mißbrauch gesicherte, feste Darreichungsform umfassend wenigstens einen Wirkstoff mit Mißbrauchspotential und wenigstens einen davon räumlich getrennten Antagonisten für diesen Wirkstoff, wobei der Wirkstoff bzw. die Wirkstoffe in wenigstens einer Untereinheit (a) und der Antagonist bzw. die Antagonisten in wenigstens einer Untereinheit (b) vorliegen und der Antagonist bzw. die Antagonisten aus der Untereinheit (b) bei bestimmungsgemäßer Applikation der Darreichungsform im Körper praktisch nicht freigesetzt wird (werden).

Eine Vielzahl von pharmazeutischen Wirkstoffen weist neben einer ausgezeichneten Wirksamkeit auf ihrem betreffenden Anwendungsgebiet auch ein Mißbrauchspotential auf, d.h. sie können von einem Mißbraucher eingesetzt werden, um Wirkungen herbeizuführen, die nicht ihrem medizinischen Bestimmungszweck entsprechen.
So werden beispielsweise Opiate, die eine exzellente Wirksamkeit bei der Bekämpfung von starken bis sehr starken Schmerzen zeigen, von Mißbrauchem häufig zum Erzielen rauschartiger, euphorisierender Zustände verwendet.

Orale Darreichungsformen, die solche Wirkstoffe mit Mißbrauchspotential enthalten, führen üblicherweise selbst bei der Einnahme mißbräuchlich hoher Mengen nicht zu dem vom Mißbraucher gewünschten Ergebnis, da die Wirkstoffe im Blut nur langsam anfluten. Um dennoch einen Mißbrauch zu ermöglichen, werden die entsprechenden Darreichungsformen vom Mißbraucher zerkleinert, z.B. gemörsert, und z.B. durch Schnupfen über die Nase appliziert. Bei einer weiteren Form des Mißbrauchs wird der Wirkstoff aus dem durch Zerkleinerung der Darreichungsform erhaltenen Pulver mit Hilfe einer vorzugsweise wäßrigen Flüssigkeit extrahiert und die resultierende Lösung, ggf. nach Filtration durch Watte oder Zellstoff, parenteral, insbesondere intravenös, appliziert. Bei diesen Formen der Applikation kommt zu einem gegenüber der oralen Applikation beschleunigten Anfluten des Wirkstoffes mit dem vom Mißbraucher gewünschten Ergebnis.

Zur Verhinderung dieser Form des Mißbrauchs wird in der WO 97/33566 eine Darreichungsform in Form eines oralen osmotischen therapeutischen Systems mit einem zweischichtigen Kern vorgeschlagen, wobei die erste, der Öffnung des Systems zugewandten Schicht des Kerns ein opioides Analgetikum und die zweite Schicht einen Antagonisten für dieses opioide Analgetikum aufweist und gleichzeitig die Push-Funktion, d.h. das Herausdrücken des Analgetikums aus der entsprechenden Schicht aus der Öffnung des Systems ausübt.

Die US 6,277,384 B1 offenbart eine Darreichungsform enthaltend eine Kombination aus einem Opioid Agonisten und einem Opioid-Antagonisten in einem bestimmten Verhältnis, welche bei der Verabreichung an eine suchtkranke Person eine negative Wirkung hervorruft.

In der US 6,228,863 wird eine Darreichungsform enthaltend eine Kombination aus einem Opioid Agonisten und einem Opioid-Antagonisten beschrieben, deren Formulierung so gewählt worden ist, daß die beiden Verbindungen jeweils nur gemeinsam aus der Darreichungsform extrahiert werden können und anschließend ein wenigstens zweistufiges Verfahren zu ihrer Separierung erforderlich ist.

Alle diese im Stande der Technik beschriebenen Darreichungsformen haben den Nachteil, daß auch bei ihrer bestimmungsgemäßen Applikation neben dem opioiden Wirkstoff jeweils auch der entsprechende Antagonist freigesetzt wird. Dies führt dazu, daß die Wirksamkeit des Opioid Agonisten beeinträchtigt wird und dessen zur zufriedenstellenden Therapie des Patienten erforderliche Menge in der Darreichungsform erhöht werden muß. Das Risiko des Auftretens unerwünschter Begleiterscheinungen wird gegenüber Darreichungsformen, die keinen Opioid-Antagonisten enthalten erhöht.
Des weiteren ist es grundsätzlich erstrebenswert, die Belastung des Patienten bei bestimmungsgemäßer Applikation einer solchen Darreichungsform nicht auch noch durch den freigesetzten Anteil an Opioid-Antagonisten weiter zu erhöhen.

Die Aufgabe der vorliegenden Erfindung bestand daher darin, eine Darreichungsform zur Verfügung zu stellen, die diese Nachteile des Standes der Technik nicht aufweist.

Diese Aufgabe wurde durch die erfindungsgemäßs und in den Ansprüchen definierte, gegen Mißbrauch gesicherte, feste Darreichungsform gelöst, die wenigstens einen Wirkstoff mit Mißbrauchspotential und wenigstens einen davon räumlich getrennten Antagonisten für diesen Wirkstoff umfaßt, wobei der Wirkstoff bzw. die Wirkstoffe in wenigstens einer Untereinheit (a) und der Antagonist bzw. die Antagonisten in wenigstens einer Untereinheit (b) vorliegen und der Antagonist bzw. die Antagonisten aus der Untereinheit (b) bei bestimmungsgemäßer Applikation der Darreichungsform im Körper praktisch nicht freigesetzt wird.

Untereinheiten im Sinne der vorliegenden Erfindung sind feste Formulierungen, die jeweils neben üblichen, dem Fachmann bekannten Hilfsstoffen nur den (die) Wirkstoff(e) oder nur den (die) Antagonist(en) aufweisen. Methoden zur Herstellung entsprechender Untereinheiten sind dem Fachmann bekannt, beispielsweise aus "Coated Pharmaceutical Dosage Forms - Fundamentals, Manufacturing Techniques, Biopharmaceutical Aspects, Test Methods and Raw Materials" von Kurt H. Bauer, K. Lehmann, Hermann P. Osterwald, Rothgang, Gerhart, 1. Auflage, 1998, Medpharm Scientific Publishers. Die entsprechende Beschreibung wird hiermit als Referenz eingeführt und gilt als Teil der Offenbarung.

Die erfindungsgemäße Darreichungsform kann in ihren jeweiligen Untereinheiten (a) bzw. (b) jeweils einen oder mehrere Wirkstoffe mit Mißbrauchspotential sowie einen oder mehrere Antagonisten aufweisen. Vorzugsweise weist die erfindungsgemäße Darreichungsform in den entsprechenden Untereinheiten jeweils nur einen Wirkstoff und nur einen Antagonisten für diesen Wirkstoff auf.

Pharmazeutische Wirkstoffe mit Mißbrauchspotential sind, ebenso wie deren einzusetzende Mengen und Verfahren zu ihrer Herstellung, dem Fachmann an sich bekannt und können als solche in Form entsprechender Derivate, insbesondere Ester oder Ether, oder jeweils in Form entsprechender physiologisch verträglicher Verbindungen, insbesondere in Form ihrer Salze oder Solvate in der erfindungsgemäßen Darreichungsform vorliegen.

Die erfindungsgemäße Darreichungsform eignet sich besonders zur Verhinderung des Mißbrauchs eines pharmazeutischen Wirkstoffs, der ausgewählt ist aus der Gruppe bestehend aus Opiaten, Opioiden, Stimulantien und weiteren Betäubungsmitteln.

Besonders geeignet ist die erfindungsgemäße Darreichungsform zur Verhinderung des Mißbrauchs von Opiaten, Opioiden sowie weitere Betäubungsmitteln, die ausgewählt sind aus der Gruppe bestehend aus N-{1-[2-(4-Ethyl-5-oxo-2-tetrazolin-1-yl)ethyl]-4-methoxymethyl-4-piperidyl}propionanilid (Alfentanil), Allylprodin, Alphaprodin, 2-Diethylaminopropiophenon (Amfepramon), (±)-α-Methylphenethylamin (Amfetamin), 2-(α-Methylphenethylamino)-2-phenylacetonitril (Amfetaminil), Anileridin, Apocodein, Benzylmorphin, Bezitramid, 17-Cyclopropylmethyl-4,5α-epopxy-7α[(S)-1-hydroxy-1,2,2-trimethyl-propyl]-6-methoxy-6,14-*endo*-ethanomorphinan-3-ol (Buprenorphin), Butorphanol, (1*S*,2*S*)-2-Amino-1-phenyl-1-propanol (Cathin / D-Norpseudoephedrin), Clonitazen, (-)-Methyl-[3β-benzoyloxy-2β(1αH,5α*H*-tropancarboxylat] (Cocain), 4,5α-Epoxy-3-methoxy-17-methyl-7-morphinen-6a-ol (Codein), Cyclorphan, Cyprenorphin, Desomorphin, Dextromoramid, (+)-(1-Benzyl-3-dimethylamino-2-methyl-1-phenylpropyl)propionat (Dextropropoxyphen), Dezocin, Diampromid, Diamorphon, 4,5α-Epoxy-3-methoxy-17-methyl-6α-morphinanol (Dihydrocodein), 4,5α-Epoxy-17-methyl-3,6a-morphinandiol (Dihydromorphin), Dimenoxadol, Dimephetamol, Dimethylthiambuten, Dioxaphetylbutyrat, Dipipanon, (6a*R,*10a*R*)-6,6,9-Trimethyl-3-pentyl-6a,7,8,10a-tetrahydro-6*H*-benzo[c]chromen-1-ol (Dronabinol), Eptazocin, Ethoheptazin, Ethylmethylthiambuten, 4,5α-Epoxy-3-ethoxy-17-methyl-7-morphinen-6α-ol (Ethylmorphin), Etonitazen, 4,5α-Epoxy-7α-(1-hydroxy-1-methylbutyl)-6-methoxy-17-methyl-6,14-*endo*-etheno-morphinan-3-ol (Etorphin), *N*-Ethyl-3-phenyl-8,9,10-trinorbornan-2-ylamin (Fencamfamin), 7-[2-(α-Methylphenethylamino)ethyl]-theophyllin) (Fenetyllin), 3-(α-Methylphenethylamino)propionitril (Fenproporex), *N*-(1-Phenethyl-4-piperidyl)propionanilid (Fentanyl), Heroin, 4,5α-Epoxy-3-methoxy-17-methyl-6-morphinanon (Hydrocodon), 4,5α-Epoxy-3-hydroxy-17-methyl-6-morphinanon (Hydromorphon), Hydroxypethidin, Isomethadon, Hydroxymethylmorphinan, 1-[4-(3-Hydroxyphenyl)-1-methyl-4-piperidyl]-1-propanon (Ketobemidon), (3*S*,6*S*)-6-Dimethylamino-4,4-diphenylheptan-3-ylacetat (Levacetylmethadol (LAAM)), (-)-6-Dimethylamino-4,4-diphenyl-3-heptanon (Levomethadon), (-)-17-Methyl-3-morphinanol (Levorphanol), Levophenacylmorphan, Lofentanil, 5-(4-Chlorphenyl)-2,5-dihydro-3*H*-imidazo[2,1-*a*]isoindol-5-ol (Mazindol), *N*-(3-Chlorpropyl)-α-methylphenethylamin (Mefenorex), Meperidin, Meptazinol, Metazocin, Methylmorphin, N,α-Dimethylphenethylamin (Metamfetamin), (±)-6-Dimethylamino-4,4-diphenyl-3-heptanon (Methadon), Methyl-[2-phenyl-2-(2-piperidyl)acetat] (Methylphenidat), 3,3-Diethyl-5-methyl-2,4-piperidindion (Methyprylon), 2-(Benzhydrylsulfinyl)acetamid (Modafinil), 4,5α-Epoxy-17-methyl-7-morphinen-3,6a-diol (Morphin), Myrophin, (±)-*trans*-3-(1,1-Dimethylheptyl)-7,8,10,10α-tetrahydro-1-hydroxy-6,6-dimethyl-6*H*-dibenzo [*b*, *d*]pyran-9(6α*H*)-on (Nabilon), Nalbuphen, Narcein, Nicomorphin, Norlevorphanol, 6-Dimethylamino-4,4-diphenyl-3-hexanon (Normethadon), Normorphin, Norpipanon, der geronnene Saft der zur Art Papaver somniferum gehörenden Pflanzen (Opium), 4,5α-Epoxy-14-hydroxy-3-methoxy-17-methyl-6-morphinanon (Oxycodon), Oxymorphon, Pflanzen und Pflanzenteile der zur Art Papaver somniferum (einschließlich der Unterart setigerum) gehörenden Pflanzen (Papaver somniferum), Papaveretum, 2-Imino-5-phenyl-4-oxazolidinon (Pernolin), 1,2,3,4,5,6-Hexahydro-6,11-dimethyl-3-(3-methyl-2-butenyl)-2,6-methano-3-benzazocin-8-ol (Pentazocin), Ethyl-(1-methyl-4-phenyl-4-piperidincarboxylat) (Pethidin), Phenadoxon, Phenomorphan, Phenazocin, Phenoperidin, Piminodin, Pholcodein, 3-Methyl-2-phenylmorpholin (Phenmetrazin), α,α-Dimethylphenethylamin (Phentermin), α-(2-Piperidyl)benzhydrylalkohol (Pipradrol), 1'-(3-Cyan-3,3-diphenylpropyl)[1,4'-bipiperidin]-4'-carboxamid (Piritramid), Profadol, Proheptazin, Promedol, Properidin, Propoxyphen, N-(1-Methyl-2-piperidinoethyl)-N-(2-pyridyl)propionamid, Methyl{3-[4-methoxycarbonyl-4-(N-phenylpropanamido)piperidino]propanoat} (Remifentanil), N-{4-Methoxymethyl-1-[2-(2-thienyl)ethyl]-4-piperidyl}propionanilid (Sufentanil), Ethyl-(2-dimethylamino-1-phenyl-3-cyclohexen-1-carboxylat) (Tilidin (cis und trans)), Tramadol, (1 R*,2R*)-3-(3-Dimethylamino-1-ethyl-2-methyl-propyl)-phenol, (1R, 2R, 4S)-2-[Dimethylamino)methyl-4-(p-fluorbenzyloxy)-1-(m-methoxyphenyl)cyclohexanol, jeweils ggf. in Form entsprechender stereoisomerer Verbindungen sowie entsprechender Derivate, insbesondere Ester oder Ether, und jeweils physiologisch verträglicher Verbindungen, insbesondere Salze und Solvate.

Die Verbindungen (1R*,2R*)-3-(3-Dimethylamino-1-ethyl-2-methyl-propyl)-phenol und (1R, 2R, 4S)-2-[Dimethylamino)methyl-4-(p-fluorbenzyloxy)-1-(m-methoxyphenyl)cyclohexanol, deren physiologisch verträgliche Verbindungen, insbesondere deren Hydrochloride, sowie Verfahren zu ihrer Herstellung sind z.B. aus EP-A-693475 bzw. EP-A.780369 bekannt. Die entsprechenden Beschreibungen werden hiermit als Referenz eingeführt und gelten als Teil der Offenbarung.

Die erfindungsgemäße Darreichungsform eignet sich auch zur Verhinderung des Mißbrauchs von Stimulanzien, bevorzugt solcher, die ausgewählt sind aus der Gruppe bestehend aus Amphetamin, Norpseudoephedrin, Methylphenidat und jeweils ggf. deren entsprechenden physiologisch verträglichen Verbindungen, insbesondere deren Basen, Salzen und Solvaten.

Geeignete Antagonisten für die jeweiligen Wirkstoffe zur Verhinderung ihres Mißbrauchs sind dem Fachmann an sich bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in üblichen, dem Fachmann bekannten Mengen in der erfindungsgemäßen Darreichungsform vorliegen.

Sofern die Untereinheit (a) als Wirkstoff ein Opiat oder eine Opioid aufweist, kommt als Antagonist in der Untereinheit (b) bevorzugt Naloxon, Naltrexon, Nalmefen, Nalid, Nalmexon, Nalorphin oder Naluphin, jeweils ggf. in Form einer entsprechenden physiologisch verträglichen Verbindung, insbesondere in Form einer Base, eines Salzes oder Solvates, zum Einsatz. Vorzugsweise weist die erfindungsgemäße Derreichungsform die entsprechenden Antagonisten in einer Menge von ≥ 10 mg, besonders bevorzugt in einer Menge von 10 bis 100 mg, ganz besonders bevorzugt in einer Menge von 10 bis 50 mg auf pro Darreichungsform, d.h. pro Dosiereinheit auf.

Weist die Untereinheit (a) als Wirkstoff ein Stimulanz auf, ist der Antagonist der Untereinheit (b) bevorzugt ein Neuroleptikum, vorzugsweise ausgewählt aus der Gruppe bestehend aus Haloperidol, Promethacin, Fluphenazin Perphenazin, Levomepromzin, Thioridazin, Perazin, Chlorpromazin, Chlorpirothixin, Melpéran Flupentexol, Prothipendyl Zotepin, Benperidol Pipomperon Melperon Bromperidol. Vorzugsweise weist die erfindungsgemäße Darreichungsform die entsprechenden Antagonisten in einer üblichen, dem Fachmann bekannten therapeutischen Dosierung, besonders bevorzugt in einer gegenüber der üblichen Dosierung verdoppelten bis verdreifachten Menge pro Dosiereinheit auf.

Ein wesentlicher Aspekt der vorliegenden Erfindung besteht darin, daß der Antagonist aus der Untereinheit bzw. den Untereinheiten (b) der erfindungsgemäßen Darreichungsform bei bestimmungsgemäßer Applikation im Körper praktisch nicht freigesetzt wird.
Der Fachmann versteht, daß die hierzu erforderlichen Formulierungsbedingungen in Abhängigkeit von dem jeweils eingesetzten Antagonisten und der Formulierung der Untereinheit (b) bzw. der Darreichungsform variieren können. Die für den jeweiligen Antagonisten optimale Formulierung kann durch einfache Vorversuche ermittelt werden.

Wird die erfindungsgemäße Darreichungsform zum Zwecke der mißbräuchlichen Einnahme des Wirkstoffes manipuliert, z.B. durch zermörsern und ggf. extrahieren des so erhaltenen Pulvers mit einem geeigneten Extraktionsmittel, wird neben dem Wirkstoff auch der Antagonist in einer Form erhalten, in der er von dem Wirkstoff nicht auf einfache Weise zu separieren ist, so daß der Wirkstoff bei der Applikation der manipulierten Darreichungsform, insbesondere bei nasaler und/oder parenteraler Verabreichung, nicht die vom Mißbraucher gewünschte Wirkung entfalten kann.

Die Formulierung der erfindungsgemäßen Darreichungsform kann in vielfältiger Art und Weise nach üblichen, dem Fachmann bekannten Methoden erfolgen, wobei die Untereinheiten (a) und (b) in der erfindungsgemäßen Darreichungsform jeweils in beliebiger räumlicher Anordnung zueinander vorliegen können, solange gewährleistet ist, daß der Antagonist im Körper praktisch nicht freigesetzt wird. Methoden zur Formulierung der Darreichungsformen sind dem Fachmann bekannt, beispielsweise aus "Coated Pharmaceutical Dosage Forms - Fundamentals, Manufacturing Techniques, Biopharmaceutical Aspects, Test Methods and Raw Materials" von Kurt H. Bauer, K. Lehmann, Hermann P. Osterwald, Rothgang. Gerhart, 1. Auflage, 1998, Medpharm Scientific Publishers. Die entsprechende Beschreibung wird hiermit als Referenz eingeführt und gilt als Teil der Offenbarung.

In einer Ausführungsform der erfindungsgemäßen Darreichungsform liegen beide Untereinheiten (a) und (b) in multipartikulärer Form vor, wobei Mikrotabletten, Mikrokapseln, Mikropellets, Granulaten, Sphäroiden, Perlen oder Pellets bevorzugt sind und sowohl für die Untereinheit (a) als auch (b) dieselbe Form, d.h. Gestaltung gewählt wird, damit keine Separierung der Untereinheiten (a) von (b) durch mechanische Auslese möglich ist. Die multipartikulären Formen weisen bevorzugt eine Größe im Bereich von 0,1 bis 3 mm, besonders bevorzugt von 0,5 bis 2 mm auf.

Die Untereinheiten (a) und (b) in multpartikulärer Form können auch bevorzugt in eine Kapsel abgefüllt, in einer Flüssigkeit oder einem Gel suspendiert oder zu einer Tablette verpreßt werden, wobei die jeweiligen Endformulierungen dergestalt erfolgen, daß die Untereinheiten (a) und (b) auch in der resultierenden Darreichungsform erhalten bleiben.

Die jeweiligen multipartikulären Untereinheiten (a) bzw (b) mit identischer Formgebung dürfen auch nicht visuell voneinander unterscheidbar sein, damit sie vom Mißbraucher nicht durch einfaches Sortieren voneinander separiert werden können. Dies kann beispielsweise durch das Aufbringen identischer Überzüge gewährleistet werden, die neben dieser Egalisierungsfunktion auch weitere Funktionen übernehmen können, wie z.B. die Retardierung eines oder mehrerer Wirkstoffe oder eine magensaftresistente Ausrüstung der jeweiligen Untereinheiten.

In einer weiteren bevorzugten Ausführungsform der vorliegenden Erfindung sind die Untereinheiten (a) und (b) jeweils schichtförmig zueinander angeordnet sind.

Bevorzugt sind hierfür die schichtförmigen Untereinheiten (a) und (b) in der erfindungsgemäßen Darreichungsform vertikal oder horizontal zueinander angeordnet, wobei jeweils auch eine oder mehrere schichtförmige Untereinheiten (a) und eine oder mehrere schichtförmige Untereinheiten (b) in der Darreichungsform vorliegen können, so daß neben den bevorzugten Schichtenfolgen (a)-(b) bzw. (a)-(b)-(a) beliebige andere Schichtenfolgen in Betracht kommen.

Ebenfalls bevorzugt ist eine erfindungsgemäße Darreichungsform, in der die Untereinheit (b) einen Kern bildet, der von der Untereinheit (a) vollständig umhüllt wird. Ein entsprechender Aufbau eignet sich bevorzugt auch für die obenstehend genannten multipartikulären Formen, wobei dann beide Untereinheiten (a) und (b) sowie eine ggf. zwischen diesen vorhandene Trennschicht (c) in ein- und derselben multipartikulären Form formuliert sind.

In einer weiteren Ausführungsform der erfindungsgemäßen Darreichungsform bildet die Untereinheit (a) einen Kern, der von der Untereinheit (b) umhüllt wird, wobei letztere wenigstens einen Kanal aufweist, der von dem Kern an die Oberfläche der Darreichungsform führt.

Zwischen einer Schicht der Untereinheit (a) und einer Schicht der Untereinheit (b) kann die erfindungsgemäße Darreichungsform jeweils eine oder mehrere, vorzugsweise eine, ggf. quellbare Trennschicht (c) zur räumlichen Trennung der Untereinheit (a) von (b) aufweisen.

Sofern die erfindungsgemäße Darreichungsform die schichtförmigen Untereinheiten (a) und (b) sowie eine ggf. vorhandene Trennschicht (c) in einer zumindest teilweise vertikalen oder horizontalen Anordnung aufweist, liegt sie bevorzugt in Form einer Tablette, eines Coextrudats oder Laminats vor.

Hierbei kann in einer besonders bevorzugten Ausführungsform die freie Oberfläche der Untereinheit (b) vollständig und ggf. zumindest ein Teil der freien Oberfläche der Untereinheit(en) (a) und ggf. zumindest ein Teil der freien Oberfläche der ggf. vorhandenen Trennschicht(en) (c) mit wenigstens einer die Freisetzung des Antagonisten verhindernden Barriereschicht (d) überzogen sein.

Ebenfalls besonders bevorzugt ist eine Ausführungsform der erfindungsgemäßen Darreichungsform, die eine vertikale oder horizontale Anordnung der Schichten der Untereinheiten (a) und (b) und wenigstens eine dazwischen angeordnete Push-Schicht (p) sowie ggf. eine Trennschicht (c) aufweist, in der sämtliche freie Oberflächen des aus den Untereinheiten (a) und (b), der Push-Schicht und der ggf. vorhandenen Trennschicht (c) bestehenden Schichtaufbaus mit einem semipermeablen Überzug (e) umhüllt sind, der für ein Freisetzungsmedium, d.h. üblicherweise eine physiologische Flüssigkeit, durchlässig, für den Wirkstoff und für den Antagonisten im wesentlichen undurchlässig ist, und wobei dieser Überzug (e) im Bereich der Untereinheit (a) wenigstens eine Öffnung zur Freisetzung des Wirkstoffes aufweist.

Eine entsprechende Darreichungsform ist dem Fachmann beispielsweise auch unter der Bezeichnung orales osmotisches therapeutisches System (OROS), ebenso wie geeignete Materialien und Verfahren zu dessen Herstellung, u.a. aus US 4,612,008, US 4,765,989 und US 4,783,337 bekannt. Die entsprechenden Beschreibungen werden hiermit als Referenz eingeführt und gelten als Teil der Offenbarung. Sofern die erfindungsgemäße Darreichungsform in Form eines OROS vorliegt, sind die Untereinheiten (a) und (b) räumlich voneinander getrennt, vorzugsweise durch eine quellbare Trennschicht, die gleichzeitig die Push-Schicht des OROS ist.

In einer weiteren Ausführungsform hat die Untereinheit (a) der erfindungsgemäßen Darreichungsform die Form einer Tablette, deren Steg und ggf. eine der beiden Grundflächen mit einer den Antagonisten enthaltenden Barrierschicht als Untereinheit (b) bedeckt ist.

Der Fachmann versteht, daß die bei der Formulierung der erfindungsgemäßen Darreichungsform jeweils zum Einsatz kommenden Hilfsstoffe der Untereinheit(en) (a) bzw. (b) sowie ggf. der vorhandenen Trennschicht(en) (c) und/oder der Barriereschicht(en) (d) in Abhängigkeit von deren Anordnung in der erfindungsgemäßen Darreichungsform, der Applikationsart sowie in Abhängigkeit von dem jeweiligen Wirkstoff und dem Antagonisten variieren. Die Materialien, die über die jeweils erforderlichen Eigenschaften verfügen sind dem Fachmann an sich bekannt.

Sofern die Freisetzung des Antagonisten aus der Untereinheit (b) der erfindungsgemäßen Darreichungsform mit Hilfe einer Umhüllung, vorzugsweise einer Barriereschicht, verhindert wird, kann die Untereinheit aus üblichen, dem Fachmann bekannten Materialien bestehen.

Ist eine entsprechende Barriereschicht (d) zur Verhinderung der Freisetzung des Antagonisten nicht vorgesehen, sind die Materialien der Untereinheiten so zu wählen, daß eine Freisetzung des Antagonisten aus der Untereinheit (b) praktisch ausgeschlossen ist.
Bevorzugt können hierzu die nachstehend aufgeführten Materialien zum Einsatz kommen, die auch für den Aufbau der Barriereschicht geeignet sind:

Bevorzugte Materialien sind solche, die ausgewählt sind aus der Gruppe bestehend aus Alkylcellulosen, Hydroxyalkylcellulosen, Glucanen, Skleroglucanen, Mannanen, Xanthanen, Copolymeren aus Poly[bis(p-carboxyphenoxy)propan und Sebacinsäure, vorzugsweise in einem Molverhältnis von 20:80 (unter der Bezeichnung Polifeprosan 20^{Θ} am Markt geführt), Carboxymethylcellulosen, Celluloseethern, Celluloseestern, Nitrocellulosen, Polymeren auf Basis von (Meth)acrylsäure sowie deren Estern, Polyamiden, Polycarbonaten, Polyalkylenen, Polyalkylenglykolen, Polyalkylenoxiden, Polyalkylenterephtalate, Polyvinylalkohole, Polyvinylether, Polyvinylester, halogenierte Polyvinyle, Polyglykolide, Polysiloxane sowie Polyurethane und deren Copolymeren.

Besonders geignete Materialien können ausgewählt werden aus der Gruppe bestehend aus Methylcellulose, Ethylcellulose, Hydroxypropylcellulose, Hydroxypropylmethylcellulose, Hydroxybutylmethylcellulose, Celluloseacetat, Cellulosepropionat (von niederem, mittlerem oder erhöhtem Molekulargewicht), Celluloseacetatpropionat, Celluloseacetatbutyrat, Celluloseacetatphtalat, Carboxymethylcellulose, Cellulosetriacetat, Natrium-Cellulosesulfat, Polymethylmethacrylat, Polyethylmethacrylat, Polybutylmethacrylat, Polyisobutylmethacrylat, Polyhexylmethacrylat. Polyisodecylmethacrylat, Polylaurylmethacrylat, Polyphenylmethacrylat, Polymethylacrylat, Polyisopropylacrylat, Polyisobutylacrylat, Polyoctadecylacrylat, Polyethylen, Polyethylen niederer Dichte. Polyethylen hoher Dichte. Polypropylen, Polyethylenglykol, Polyethylenoxid, Polyethylenterephtalat, Polyvinylalkohol, Polyvinylisobutylether, Polyvinylacetat und Polyvinylchlorid.

Besonders geeignete Copolymere können ausgewählt werden aus der Gruppe bestehend aus Copolymeren aus Butylmethacrylat und Isobutylmethacrylat, Copolymeren aus Methylvinylether und Maleinsäure mit erhöhtem Molekulargewicht, Copolymeren aus Methylvinylether und Maleinsäuremonoethylester, Copolymeren aus Methylvinylether und Maleinsäureanhydrid sowie Copolymeren aus Vinylalkohol und Vinylacetat.

Weitere, zur Formulierung der Barriereschicht besonders geeignete, biologisch abbaubare Materialien sind Stärke gefülltes Polycaprolacton (WO98/20073), aliphatische Polyesteramide (DE 19 753 534 A1, DE 19 800 698 A1, EP 0 820 698 A1), aliphatische und aromatische Polyesterurethane (DE 19822979), Polyhydroxyalkanoate, insbesondere Polyhydroxybutyrate, Polyhydroxyvaleriate), Casein (DE 4 309 528), Polylactide und Copolylactide (EP 0 980 894 A1). Die entsprechenden Beschreibungen werden hiermit als Referenz eingeführt und gelten als Teil der Offenbarung.

Ggf. können die vorstehend genannten Materialien mit weiteren üblichen, dem Fachmann bekannten Hilfsstoffen, vorzugsweise ausgewählt aus der Gruppe bestehend aus Glycerylmonostearat, halbsynthetischen Triglyceridderivaten, halbsynthetischen Glyceriden, hydriertem Rizinusöl, Glycerylpalmitostearat, Glycerylbehenat, Polyvinylpyrrolidon, Gelatine, Magnesiumstearat, Stearinsäure, Natriumstearat, Talkum, Natriumbenzoat, Borsäure und kolloidalem Silica, Fettsäuren, substituierten Triglyceriden, Glyceriden, Polyoxyalkylenglykolen und deren Derivate abgemischt werden.

Sofern die erfindungsgemäße Darreichungsform eine Trennschicht (c) aufweist, kann diese, ebenso wie die nicht von einer Barriereschicht umhüllte Untereinheit (b) vorzugsweise aus den vorstehend, für die Barriereschicht beschriebenen Materialien bestehen. Der Fachmann versteht, daß auch über die Dicke der Trennschicht jegliche Freisetzung des Antagonisten aus der jeweiligen Untereinheit verhindert werden kann.

Die erfindungsgemäße Darreichungsform zur oralen Verabreichung eines oder mehrerer Wirkstoffe eignet sich besonders zur Verhinderung eines oralen, nasalen und/oder parenteralen Mißbrauchs solcher Wirkstoffe.

Dabei können auch ein oder mehrere Wirkstoffe zumindest teilweise in retardierter Form vorliegen, wobei die Retardierung mit Hilfe von üblichen, dem Fachmann bekannten Materialien und Verfahren erzielt werden kann, beispielsweise durch Einbetten des Wirkstoffes in eine retardierende Matrix oder durch das Aufbringen eines oder mehrerer retardierender Überzüge. Die Wirkstoffabgabe muß aber so gesteuert sein, daß bei bestimmungsgemäßer Applikation der Darreichungsform der Antagonist im Körper praktisch nicht freigesetzt wird.

Die erfindungsgemäße Darreichungsfom ist zur oralen Applikation vorgesehen und kann bevorzugt auch einen magensaftresistenten Überzug aufweisen, der sich in Abhängigkeit vom pH-Wert der Freisetzungsumgebung auflöst. Durch diesen Überzug kann erreicht werden, daß die erfindungsgemäße Darreichungsform den Magentrakt unaufgelöst passiert und der Wirkstoff erst im Darmtrakt zur Freisetzung gelangt. Vorzugsweise löst sich der magensaftresistente Überzug bei einem pH-Wert zwischen 5 und 7,5 auf. Auch bei dieser Ausführungsform ist die Untereinheit (b) selbstverständlich so zu formulieren, daß der Antagonist im Körper praktisch nicht freigesetzt wird.

Entsprechende Materialien und Verfahren zur Retardierung von Wirkstoffen sowie zum Aufbringen magensaftresistenter Überzüge werden beispielsweise in "Coated Pharmaceutical Dosage Forms - Fundamentals, Manufacturing Techniques, Biopharmaceutical Aspects, Test Methods and Raw Materials" von Kurt H. Bauer, K. Lehmann, Hermann P. Osterwald, Rothgang, Gerhart, 1. Auflage, 1998, Medpharm Scientific Publishers beschrieben. Die entsprechende Beschreibung wird hiermit als Referenz eingeführt und gilt als Teil der Offenbarung.

Die erfindungsgemäßen Darreichungsformen haben den Vorteil, daß sie gegen nasalen und/oder parenteralen geschützt sind, ohne daß bei bestimmungsgemäßer Applikation eine unnötige Belastung des zu therapierenden Patienten oder eine Verminderung der Wirksamkeit des jeweiligen Wirkstoffes zu befürchten ist. Sie lassen sich einfach und vergleichsweise kostengünstig produzieren.

Im folgenden wird die Erfindung anhand von Beispielen erläutert. Diese Erläuterungen sind lediglich beispielhaft und schränken den allgemeinen Erfindungsgedanken nicht ein.

### Beispiele:

Die nachstehenden Mengenangaben beziehen sich jeweils auf eine Darreichungsform. Eine Charge eines Herstellgangs bestand aus 1000 Darreichungsformen.

### Beispiel 1

### Manteltabletten

### Kern

| | |
|---|---|
| Naltrexonhydrochlorid | 50 mg |
| Hydriertes Rizinusöl (Cutina HR) | 50 mg |

Naltrexonhydrochlorid und fein gepulvertes hydriertes Rizinusöl wurden gemischt und auf einer Tablettenpresse zu runden, bikonvexen Tabletten mit 6,5 mm Durchmesser verpreßt.

### Mantel

| | |
|---|---|
| Morphinsulfat Pentahydrat | 60 mg |
| Methylhydroxypropylcellulose 100 000 mPas (Metolose 90 SH 100 000, ShinEtsu) | 100 mg |
| Mikrokristalline Cellulose (Avicel PH 102) | 165 mg |
| Lactose Monohydrat | 165 mg |
| Magnesiumstearat | 5 mg |
| Kolloidales Siliciumdioxid | 5 mg |

Alle Mantelbestandteile wurden gemischt, in einer Tablettenpresse mit Werkzeug für 13 mm bikonvexe Tabletten werden ca. 250 mg der Mischung in die Tablettenmatrize gefüllt, der 6,5 mm Kern zentriert eingelegt, die restlichen 250 mg der Mantelmischung zugefüllt und der Mantel um den Kern gepreßt.

### Beispiel 2

### Manteltabletten

### Kern

| | |
|---|---|
| Naltrexonhydrochlorid | 50 mg |
| Hydriertes Rizinusöl (Cutina HR) | 50 mg |

Naltrexonhydrochlorid und fein gepulvertes Hydriertes Rizinusöl wurden gemischt und auf einer Tablettenpresse zu runden, bikonvexen Tabletten mit 6,5 mm Durchmesser verpreßt.

### Mantel

| | |
|---|---|
| Oxycodon-Hydrochlorid | 30 mg |
| Srühgetrocknete Lactose | 300 mg |
| Eudragit RSPM | 70 mg |
| Stearylakohol | 115 mg |
| Magnesiumstearat | 5 mg |
| Talkum | 10 mg |

Oxycodon-Hydrochlorid, sprühgetrocknete Lactose und Eudragit RSPM wurden in einem geeigneten Mischer ca. 5 min innig miteinander vermischt. Während des Mischens wurde die Mischung mit einer solchen Menge Gereinigtem Wasser granuliert, daß sich eine feuchte granulierte Masse bildet. Das so erhaltene Granulat wurde in der Wirbelschicht bei 60°C getrocknet und durch ein 2,5 mm Sieb gesiebt. Anschließend wurde das Granulat erneut wie vor beschrieben getrocknet und durch ein 1,5 mm Sieb gesiebt. Der Stearylakohol wurde bei 60-70°C geschmolzen und in einem Mischer zu dem Granulat gegeben. Nach dem Abkühlen wurde die Masse zusammen mit Magnesiumstearat und Talkum durch ein 1,5 mm Sieb gesiebt. Vom so erhaltenen Granulat wurde in einer Tablettenpresse mit Werkzeug für 13 mm bikonvexe Tabletten ca. 265 mg der Mischung in die Tablettenmatrize gefüllt, der 6,5 mm Kern zentriert eingelegt, die restlichen 265 mg der Mantelmischung zugefüllt und der Mantel um den Kern gepreßt.

### Beispiel 3

### Manteltabletten

### Kern

| | |
|---|---|
| Naloxonhydrochlorid-Dihydrat | 20 mg |
| Sprühgetrocknete Lactose | 76 mg |
| Magnesiumstearat | 2 mg |
| Kolloidales Siliciumdioxid | 2 mg |

Alle Bestandteile wurden gemischt und auf einer Tablettenpresse zu runden, bikonvexen Tabletten mit 6,5 mm Durchmesser verpreßt.

### Überzug auf Kern

| | |
|---|---|
| Celluloseacetat mit 39,8 % Acetat | 9,5 mg |
| Macrogol 3350 | 0,5 mg |

Die Überzugsbestandtelle wurden in einem Aceton-Wasser Gemisch (95:5 Gewichtsteile) gelöst und auf die Kerne gesprüht.

### Mantel

| | |
|---|---|
| Morphinsulfat Pentahydrat | 60 mg |
| Methylhydroxypropylcellulose 100 000 mPas (Metolose 90 SH 100 000, ShinEtsu) | 100 mg |
| Mikrokristalline Cellulose (Avicel PH 102) | 165 mg |
| Lactose Monohydrat | 165 mg |
| Magnesiumstearat | 5 mg |
| Kolloidales Siliciumdioxid | 5 mg |

Alle Mantelbestandteile wurden gemischt, in einer Tablettenpresse mit Werkzeug für 13 mm bikonvexe Tabletten wurden ca. 250 mg der Mischung in die Tablettenmatrize gefüllt, der mit Celluloseacetat überzogene Kern zentriert eingelegt, die restlichen 250 mg der Mantelmischung zugefüllt und der Mantel um den Kern gepreßt.

### Beispiel 4

### Multipartikuläre Form

### Antagonisten Pellets:

| | |
|---|---|
| Naltrexonhydrochlorid | 50 mg |
| Lactose | 15 mg |
| Mikrokristalline Cellulose PH101 | 30 mg |
| Niedrigsubstituierte Hydroxypropylcellulose (LH31, Shin-Etsu) | 5 mg |

Alle Bestandteile wurden in einem geeigneten Mischer ca. 5 min innig miteinander vermischt. Während des Mischens wurde die Mischung mit einer solchen Menge Gereinigtem Wasser granuliert, daß sich eine feuchte granulierte Masse bildete. Das so erhaltene Granulat wurde in einem Nica-Extruder durch eine Matrize mit Extrusionsöffnungen von 1 mm extrudiert, 5 min auf einem Spheronizer gerundet, in der Wirbelschicht bei 60°C getrocknet und mittels eines 1,5 mm und eines 0,5 mm Siebs klassiert.

### Überzug auf Antagonisten Pellets

| | |
|---|---|
| Celluloseacetat mit 39,8 % Acetat | 9,5 mg |
| Macrogol 3350 | 0,5 mg |
| Titandioxid | 0,5 mg |

| | |
|---|---|
| Mengenangaben pro 100 mg Antagonistenpellets | |

Celluloseacetat und Macrogol wurden in einem Aceton-Wasser Gemisch (95:5 Gewichtsteile) gelöst, Titandioxid wird in der Mischung dispergiert und die Kerne in einer Wirbelschichtanlage mit der Suspension besprüht bis die Masse der überzogenen Pellets 110% des Gewichts der eingesetzten nicht überzogenen Pellets betrug.

### Analgetika Pellets

| | |
|---|---|
| Nonpareils 0,5 mm (Saccharose-Maisstärke Starter Pellets, Fa. Werner) | 50 mg |
| Morphinsulfat Pentahydrat | 60 mg |
| Povidon K 30 | 30 mg |
| Talkum | 10 mg |

Morphinsulfat und Povidon wurden in Gereinigtem Wasser gelöst und Talkum wird in der Lösung dispergiert. Die Suspension wurde bei 60°C auf die Nonpareils aufgesprüht und getrocknet. Die Pellets wurden mittels eines 1,5 mm und eines 0,5 mm Siebs klassiert.

### Überzug auf Analgetika Pellets

| | |
|---|---|
| Ethylcellulose Dispersion (Aquacoat ECD30, FMC Corporation) | 10,0 mg |
| Glycerolmonostearat | 2,0 mg |
| Talkum | 2,0 mg |
| Titandioxid | 1,0 mg |

| | |
|---|---|
| Mengenangaben pro 150 mg Analgetica Pellets, Ethylcellulosegewichtsangabe als Trockenmasse aus der 30%igen Dispersion des Handelsprodukts. | |

Ethylcellulose Dispersion wurde 1:0,5 mit Gereinigtem Wasser gemischt und das Glycerolmonostearat unter mindestens zwei stündigem Rühren eingearbeitet. Talkum und Titandioxid wurden in 0,5 Teilen Wasser (Berechnungsbasis aus der 1:0,5 Mischung der Ethylcellulose Dispersion) dispergiert und mit der Ethylcellulosedispersion gemischt. Die Analgetica Pellets wurden in einer Wirbelschichtanlage mit der Dispersion besprüht bis die Masse der überzogenen Pellets 110% des Gewichts der eingesetzten nicht überzogenen Pellets betrug.

### Endformulierung in Kapseln

Pro Kapsel wurden jeweils 110 mg überzogene Pellets enthaltend Antagonisten und 165 mg überzogene Analgetica Pellets gemischt und in Hartgelatinekapseln der Größe 1 abgefüllt.

### Beispiel 5:

### Multipartikuläre Form

### Antagonisten Pellets:

| | |
|---|---|
| Naloxonhydrochlorid-Dihydrat | 20 mg |
| Lactose | 7 mg |
| Mikrokristalline Cellulose PH101 | 20 mg |
| Niedrigsubstituierte Hydroxypropylcellulose (LH31, Shin-Etsu) | 3 mg |

Alle Bestandteile wurden in einem geeigneten Mischer ca. 5 min innig miteinander vermischt. Während des Mischens wurde die Mischung mit einer solchen Menge Gereinigtem Wasser granuliert, daß sich eine feuchte granulierte Masse bildete. Das so erhaltene Granulat wurde in einem Nica-Extruder durch eine Matrize mit Extrusionsöffnungen von 1 mm extrudiert, 5 min auf einem Spheronizer gerundet, in der Wirbelschicht bei 60°C getrocknet und mittels eines 1,5 mm und eines 0,5 mm Siebs klassiert.

### Überzug auf Antagonisten Pellets

| | |
|---|---|
| Celluloseacetat mit 39,8 % Acetat | 9,5 mg |
| Macrogol 3350 | 0,5 mg |
| Titandioxid | 0,5 mg |

| | |
|---|---|
| Mengenangaben pro 100 mg Antagonistenpellets | |

Celluloseacetat und Macrogol wurden in einem Aceton-Wasser Gemisch (95:5 Gewichtsteile) gelöst, Titandioxid wird in der Mischung dispergiert und die Kerne in einer Wirbelschichtanlage mit der Suspension besprüht bis die Masse der überzogenen Pellets 110% des Gewichts der eingesetzten nicht überzogenen Pellets betrug.

### Analgetika Pellets

| | |
|---|---|
| Nonpareils 0,5 mm (Saccharose-Maisstärke Starter Pellets, Fa. Werner) | 50 mg |
| Morphinsulfat Pentahydrat | 60 mg |
| Povidon K 30 | 30 mg |
| Talkum | 10 mg |

Morphinsulfat und Povidon wurden in Gereinigtem Wasser gelöst und Talkum wird in der Lösung dispergiert. Die Suspension wurde bei 60°C auf die Nonpareils aufgesprüht und getrocknet. Die Pellets wurden mittels eines 1,5 mm und eines 0,5 mm Siebs klassiert.

### Überzug auf Analgetika Pellets

| | |
|---|---|
| Ethylcellulose Dispersion (Aquacoat ECD30, FMC Corporation) | 10,0 mg |
| Glycerolmonostearat | 2,0 mg |
| Talkum | 2,0 mg |
| Titandioxid | 1,0 mg |

| | |
|---|---|
| Mengenangaben pro 150 mg Analgetica Pellets, Ethylcellulosegewichtsangabe als Trockenmasse aus der 30%igen Dispersion des Handelsprodukts. | |

Ethylcellulose Dispersion wurde 1:0,5 mit Gereinigtem Wasser gemischt und das Glycerolmonostearat unter mindestens zwei stündigem Rühren eingearbeitet. Talkum und Titandioxid wurden in 0,5 Teilen Wasser (Berechnungsbasis aus der 1:0,5 Mischung der Ethylcellulose Dispersion) dispergiert und mit der Ethylcellulosedispersion gemischt. Die Analgetica Pellets wurden in einer Wirbelschichtanlage mit der Dispersion besprüht bis die Masse der überzogenen Pellets 110% des Gewichts der eingesetzten nicht überzogenen Pellets betrug.

### Endformulierung in Kapseln

Pro Kapsel wurden jeweils 55 mg überzogene Pellets enthaltend Antagonisten und 165 mg überzogene Analgetica Pellets gemischt und in Hartgelatinekapseln der Größe 2 abgefüllt.

### Beispiel 6

### Orales osmotisches therapeutisches System

### Wirkstoffschicht

| | |
|---|---|
| Morphinsulfat Pentahydrat | 125 mg |
| Macrogol 200 000 | 280 mg |
| Povidon (MG_{N} 40 000) | 26 mg |
| Magnesiumstearat | 4 mg |

Morphinsulfat und Macrogol wurden in einem Planetenmischer trocken gemischt und anschließend unter langsamer Zugabe einer Lösung des Povidon in 115 mg Ethanol zu einer feuchten Masse angeteigt, die dann durch ein 0,8 mm Sieb getrieben wurden. Nach 24 Stunden Trocknen bei Raumtemperatur in einem Abzug wurden die Partikel zusammen mit dem Magnesiumstearat durch ein 1,0 mm Sieb getrieben und in einem Containermischer gemischt.

### Push-Schicht

| | |
|---|---|
| Methylhydroxypropylcellulose 6 mPas | 13 mg |
| Natriumchlorid | 80 mg |
| Macrogol 7 000 000 | 166 mg |
| Magnesiumstearat | 1 mg |

Natriumchlorid, Macrogol und die Hälfte der Methylhydroxypropylcellulose wurden in einem Wirbelschichtgranulator 3 Minuten trocken gemischt und anschließend durch Aufsprühen einer Lösung der zweiten Hälfte der Methylhydroxypropylcellulose in 75 mg unter Zufuhr von Warmluft granuliert und getrocknet. Das Granulat wurde anschließend zusammen mit dem Magnesiumstearat durch ein 2,5 mm Sieb in einer Comil getrieben.

### Antagonistenschicht

| | |
|---|---|
| Naltrexonhydrochlorid | 50 mg |
| Hydriertes Rizinusöl (Cutina HR) | 60 mg |
| Lactose | 20 mg |

Naltrexonhydrochlord und hydriertes Rizinusöl wurden in einer Tablettenpresse mit einem 10 mm Vorpreßstempel zu Preßlingen von ca. 250 mg vorgepreßt. Anschließend wurden die Vorpreßlinge mittels eines Brechers und eines Siebes von 1,0 mm zerkleinert.

### Herstellung der 3-Schichttabletten

Pro Tablette wurden 100 mg des Granulates der Antagonistenschicht, 260 mg der Push-Schicht und 435 mg der Wirkstoffschicht nacheinander in die Matrize einer geeigneten Tablettenpresse gefüllt und zu einer 3-Schichttablette verpreßt.

### Überzug auf Kern

| | |
|---|---|
| Celluloseacetat mit 39,8 % Acetat | 38 mg |
| Macrogol 3350 | 2 mg |

Die Überzugsbestandteile wurden in einem Aceton-Wasser Gemisch (95:5 Gewichtsteile) als 3,8%ige Lösung gelöst und auf die Kerne gesprüht. Durch den Überzug wurden zwei Löcher mit einem Durchmesser von 0,75 mm gebohrt um die Wirkstoffschicht mit der äußeren Umgebung des Systems zu verbinden.

### Beispiel 7

### Orales osmotisches therapeutisches System

Die Herstellung erfolgte analog zu Beispiel 6 mit dem Unterschied, daß die Antagonistenschicht die folgende Zusammensetzung aufwies:

| | |
|---|---|
| Naloxonhydrochlorid-Dihydrat | 60 mg |
| Hydriertes Rizinusöl (Cutina HR) | 40 mg |
| Lactose | 20 mg |

Naloxonhydrochlorid-Dihydrat, Hydriertes Rizinusöl und Lactose wurden in einer Tablettenpresse mit einem 10 mm Vorpreßstempel zu Preßlingen von ca. 250 mg vorgepreßt. Anschließend werden die Vorpreßlinge mittels eines Brechers und eines Siebes von 1,0 mm zerkleinert.

Alle übrigen Herstellschritte erfolgen wie in Beispiel 6 dargestellt.

## Patentansprüche

1. Gegen Mißbrauch gesicherte, orale, feste Darreichungsform umfassend wenigstens einen Wirkstoff mit Mißbrauchspotential und wenigstens einen davon räumlich getrennte Antagonisten für diesen Wirkstoff, wobei der Wirkstoff bzw. die Wirkstoffe in wenigstens einer Untereinheit (a) und der Antagonist bzw. die Antagonisten in wenigstens einer Untereinheit (b) vorliegen und der Antagonist bzw. die Antagonisten aus der Untereinheit (b) bei bestimmungsgemäßer Applikation der Darreichungsform im Körper praktisch nicht freigesetzt wird (werden), **dadurch gekennzeichnet, dass**
(X) beide der Untereinheiten (a) und (b) jeweils in multipartikulärer Form, wobei die jeweiligen multipartikulären Formen der Untereinheit (a) bzw. (b) weitgehend identisch gestaltet und visuell nicht unterscheidbar sind, vorliegen ggf. in Kapseln abgefüllt oder in einer Flüssigkeit oder einem Gel suspendiert, oder
(Y) die Untereinheit (a) einen Kern bildet, der von der Untereinheit (b) umhüllt wird, wobei diese Umhüllung wenigstens einen Kanal aufweist, der von dem Kern an die Oberfläche der Darreichungsform führt, oder
(Z) die Untereinheit (a) die Form einer Tablette hat, deren Steg und ggf. eine der beiden Grundflächen mit wenigstens einer den Antagonisten enthaltenden Barrierschicht (b) bedeckt ist.

2. Darreichungsform gemäß Anspruch 1, **dadurch gekennzeichnet, daß** der Wirkstoff bzw. die Wirkstoffe pharmazeutische Wirkstoffe aus der Gruppe bestehend aus Opiaten, Opioiden, Stimulantien und weiteren Betäubungsmitteln ausgewählt worden sind.

3. Darreichungsform gemäß Anspruch 2, **dadurch gekennzeichnet, daß** der Wirkstoffe bzw. die Wirkstoffe ausgewählt worden sind aus der Gruppe bestehend aus N-{1-[2-(4-Ethyl-5-oxo-2-tetrazolin-1-yl)ethyl]-4-methoxymethyl-4-piperidyl}propionanilid (Alfentanil), Allylprodin, Alphaprodin, 2-Diethylaminopropiophenon (Amfepramon), (±)-α-Methylphenethytamin (Amfetamin), 2-(α-Methylphenethylamino)-2-phenylacetonitril (Amfetaminil), Anileridin, A_{pocodein}, Benzylmorphin, Bezitramid, 17-Cyclopropylmethyl-4,5α-epopxy-7α[(S)-1-hydroxy-1,2,2-trimethyl-propyl]-6-methoxy-6,14-*endo-*ethanomorphinan-3-ol (Buprenorphin), Butorphanol, (1S,2S)-2-Amino-1-phenyl-1-propanol (Cathin / D-Norpseudoephedrin), Clonitazen, (-)-Methyl-[3β-benzoyloxy-2β(1αH,5α*H*)-tropancarboxylat] (Cocain), 4,5α-Epoxy-3-methoxy-17-methyl-7-morphinen-6α-ol (Codein), Cyclorphan, Cyprenorphin, Desomorphin, Dextromoramid, (+)-(1-Benzyl-3-dimethylamino-2-methyl-1-phenylpropyl)propionat (Dextropropoxyphen), Dezocin, Diampromid, Diamorphon, 4,5α-Epoxy-3-methoxy-17-methyl-6α-morphinanol (Dihydrocodein), 4,5α-Epoxy-17-methyl-3,6a-morphinandiol (Dihydromorphin), Dimenoxadol, Dimephetamol, Dimethylthiambuten, Dioxaphetylbutyrat, Dipipanon, (6aR,10aR)-6,6,9-Trimethyl-3-pentyl-6a,7,8,10a-tetrahydro-6*H*-benzo[c]chromen-1-ol (Dronabinol), Eptazocin, Ethoheptazin, Ethylmethylthiambuten, 4,5α-Epoxy-3-ethoxy-17-methyl-7-morphinen-6α-ol (Ethylmorphin), Etonitazen, 4,5α-Epoxy-7α-(1-hydroxy-1-methylbutyl)-6-methoxy-17-methyl-6,14-*endo*-etheno-morphinan-3-ol (Etorphin), *N*-Ethyl-3-phenyl-8,9,10-trinorboman-2-ylamin (Fencamfamin), 7-[2-(α-Methylphenethylamino)ethyl]-theophyllin) (Fenetyllin), 3-(α-Methylphenethylamino)propionitril (Fenproporex), *N*-(1-Phenethyl-4-piperidyl)propionanilid (Fentanyl), Heroin, 4,5α-Epoxy-3-methoxy-17-methyl-6-morphinanon (Hydrocodon), 4,5α-Epoxy-3-hydroxy-17-methyl-6-morphinanon (Hydromorphon), Hydroxypethidin, Isomethadon, Hydroxymethylmorphinan, 1-[4-(3-Hydroxyphenyl)-1-methyl-4-piperidyl]-1-propanon (Ketobemidon), (3*S*,6*S*)-6-Dimethylamino-4,4-diphenylheptan-3-ylacetat (Levacetylmethadol (LAAM)), (-)-6-Dimethylamino-4,4-diphenyl-3-heptanon (Levomethadon), (-)-17-Methyl-3-morphinanol (Levorphanol), Levophenacylmorphan, Lofentanil, 5-(4-Chlorphenyl)-2,5-dihydro-3*H*-imidazo[2,1-*a*]isoindol-5-ol (Mazindol), *N*-(3-Chlorpropyl)-α-methylphenethylamin (Mefenorex), Meperidin, Meptazinol, Metazocin, Methylmorphin, N,α-Dimethylphenethylamin (Metamfetamin), (±)-6-Dimethylamino-4,4-diphenyl-3-heptanon (Methadon), Methyl-[2-phenyl-2-(2-piperidyl)acetat] (Methylphenidat), 3,3-Diethyl-5-methyl-2,4-piperidindion (Methyprylon), 2-(Benzhydrylsulfinyl)acetamid (Modafinil), 4,5α-Epoxy-17-methyl-7-morphinen-3,6α-diol (Morphin), Myrophin, (±)-*trans*-3-(1,1-Dimethylheptyl)-7,8,10,10α-tetrahydro-1-hydroxy-6,6-dimethyl-6*H*-dibenzo [*b*, *d*]pyran-9(6α*H*)-on (Nabilon), Nalbuphen, Narcein, Nicomorphin, Norlevorphanol, 6-Dimethylamino-4,4-diphenyl-3-hexanon (Normethadon), Normorphin, Norpioanon, der geronnene Saft der zur Art Papaver somniferum gehörenden Pflanzen (Opium), 4,5α-Epoxy-14-hydroxy-3-methoxy-17-methyl-6-morphinanon (Oxycodon), Oxymorphon, Pflanzen und Pflanzenteile der zur Art Papaver somniferum (einschließlich der Unterart setigerum) gehörenden Pflanzen (Papaver somniferum), Papaveretum, 2-Imino-5-phenyl-4-oxazolidinon (Pemolin), 1,2,3,4,5,6-Hexahydro-6,11-dimethyl-3-(3-methyl-2-butenyl)-2,6-methano-3-benzazocin-8-ol (Pentazocin), Ethyl-(1-methyl-4-phenyl-4-piperidincarboxylat) (Pethidin), Phenadoxon, Phenomorphan, Phenazocin, Phenoperidin, Piminodin, Pholcodein, 3-Methyl-2-phenylmorpholin (Phenmetrazin), α,α-Dimethylphenethylamin (Phentermin), α-(2-Piperidyl)benzhydrylalkohol (Pipradrol), 1'-(3-Cyan-3,3-diphenylpropyl)[1,4'-bipiperidin]-4'-carboxamid (Piritramid), Profadol, Proheptazin, Promedol, Properidin, Propoxyphen, N-(1-Methyl-2-piperidinoethyl)-N-(2-pyridyl)propionamid, Methyl{3-[4-methoxycarbonyl-4-(*N-*phenylpropanamido)piperidino]propanoat} (Remifentanil), *N*-(4-Methoxymethyl-1-[2-(2-thienyl)ethyl]-4-piperidyl}propionanilid (Sufenfanil), Ethyl-(2-dimethylamino-1-phenyl-3-cyclohexen-1-carboxylat) (Tilidin (cis und trans)), Tramadol, (1R*,2R*)-3-(3-Dimethylamino-1-ethyl-2-methyl-propyl)-phenol, (1R, 2R, 4S)-2-[Dimethylamino)methyl-4-(p-fluorbenzyloxy)-1-(m-methoxyphenyl)cyclohexanol, jeweils ggf. in Form entsprechender stereoisomerer Verbindungen sowie entsprechender Derivate, insbesondere Ester oder Ether, und jeweils physiologisch verträglicher Verbindungen, insbesondere Salze und Solvate

4. Darreichungsform gemäß Anspruch 2 oder 3, **dadurch gekennzeichnet, daß** das Stimulanz ausgewählt worden ist aus der Gruppe bestehend aus Amphetamin, Norpseudoephedrin, Methylphenidat und jeweils ggf. deren entsprechenden physiologisch verträglichen Verbindungen, insbesondere deren Basen, Salzen und Solvaten.

5. Darreichungsform gemäß einem der Ansprüche 2 bis 4, **dadurch gekennzeichnet, daß** als Antagonist für ein Opiat oder Opioid ein Antagonist ausgewählt aus der Gruppe bestehend aus Naloxon, Naltrexon, Nalmefen, Nalid, Nalmexon, Nalorphin, Naluphin und jeweils entsprechender physiologisch verträglicher Verbindungen, insbesondere Basen, Salze und Solvate, zum Einsatz kommt.

6. Darreichungsform gemäß einem der Ansprüche 2 bis 5, **dadurch gekennzeichnet, daß** als Antagonist für ein Stimulanz ein Neuroleptikum, vorzugsweise ausgewählt aus der Gruppe bestehend aus Haloperidol, Promethacin, Fluphenazin, Perphenazin, Levomepromazin, Thioridazin, Perazin, Chlorpromazin, Chlorprothixin, zuclopentixol, Flupentexol, Prothipendyl, Zotepin, Benperidol, Pipamperon, Melperon, und Bromperidol zum Einsatz kommt.

7. Darreichungsform gemäß einem der Ansprüche 1 bis 6, **dadurch gekennzeichnet. daß** sie wenigstens einen Wirkstoff zumindest teilweise in retardierter Form aufweist.

8. Darreichungform gemäß einem der Ansprüche 1 bis 7, **dadurch gekennzeichnet**, daß-sie wenigstens einen magensaftresistenten Überzug aufweist.

## Claims

1. An abuse-proofed, solid dosage form comprising at least one active ingredient with potential for abuse and at least one antagonist for this active ingredient spatially separate therefrom, wherein the active ingredient or active ingredients is/are present in at least one subunit (a) and the antagonist or antagonists are present in at least one subunit (b) and, in the event of correct administration of the dosage form, the antagonist or antagonists is/are practically not released in the body from subunit (b), **characterised in that**
(X) both of the subunits (a) and (b) in each case assume multiparticulate form, wherein the respective multiparticulate forms of subunit (a) and (b) are largely identically shaped and visually indistinguishable from one another, optionally packaged in capsules or suspended in a liquid or a gel, or
(Y) subunit (a) forms a core, which is enclosed by subunit (b), wherein this enclosure comprises at least one channel which leads from the core to the surface of the dosage form, or
(Z) subunit (a) assumes the form of a tablet, the edge face and optionally one of the two main faces of which is covered with a barrier layer (b) containing the antagonist.

2. A dosage form according to claim 1, **characterised in that** the active ingredient or active ingredients are pharmaceutical active ingredients which have been selected from the group consisting of opiates, opioids, stimulants and further narcotics.

3. A dosage form according to claim 2, **characterised in that** the active ingredient or active ingredients have been selected from the group consisting of N-{1-[2-(4-ethyl-5-oxo-2-tetrazolin-1-yl)ethyl]-4-methoxymethyl-4-piperidyl}propionanilide (alfentanil), allylprodine, alphaprodine, 2-diethylaminopropiophenone (amfepramone), (±)-α-methylphenethylamine (amphetamine), 2-(α-methylphenethylamino)-2-phenylacetonitrile (amphetaminil), anileridine, apocodeine, benzylmorphine, bezitramide, 17-cyclopropylmethyl-4,5α-epoxy-7α[(S)-1-hydroxy-1,2,2-trimethyl-propyl]-6-methoxy-6,14-*endo*-ethanomorphinan-3-ol (buprenorphine), butorphanol, (1*S*,2*S*)-2-amino-1-phenyl-1-propanol (cathine/D-norpseudoephedrine), clonitazene, (-)-methyl-[3β-benzoyloxy-2β(1α*H*, 5α*H*) - tropane carboxylate] (cocaine), 4,5α-epoxy-3-methoxy-17-methyl-7-morphinen-6α-ol (codeine), cyclorphan, cyprenorphine, desomorphine, dextromoramide, (+)-(1-benzyl-3-dimethylamino-2-methyl-1-phenylpropyl) propionate (dextropropoxyphene), dezocine, diampromide, diamorphone, 4,5α-epoxy-3-methoxy-17-methyl-6α-morphinanol (dihydrocodeine), 4,5α-epoxy-17-methyl-3,6a-morphinandiol (dihydromorphine), dimenoxadol, dimephetamol, dimethylthiambutene, dioxaphetyl butyrate, dipipanone, (6a*R*, 10a*R*) -6,6,9-trimethyl-3-pentyl-6a,7,8,10a-tetrahydro-6*H-*benzo[c]chromen-1-ol (dronabinol), eptazocine, ethoheptazine, ethylmethylthiambutene, 4,5α-epoxy-3-ethoxy-17-methyl-7-morphinen-6α-ol (ethylmorphine), etonitazene, 4,5α-epoxy-7α-(1-hydroxy-1-methylbutyl)-6-methoxy-17-methyl-6,14-*endo*-etheno-morphinan-3-ol (etorphine), *N*-ethyl-3-phenyl-8,9,10-trinorbornan-2-ylamine (fencamfamine), 7-[2-(α-methylphenethylamino)-ethyl]-theophylline) (fenethylline), 3-(α-methyl-phenethylamino)propionitrile (fenproporex), *N*-(1-phenethyl-4-piperidyl)propionanilide (fentanyl), heroin, 4,5α-epoxy-3-methoxy-17-methyl-6-morphinanone (hydrocodone), 4,5α-epoxy-3-hydroxy-17-methyl-6-morphinanone (hydromorphone), hydroxypethidine, isomethadone, hydroxymethylmorphinan, 1-[4-(3-hydroxyphenyl)-1-methyl-4-piperidyl]-1-propanone (ketobemidone), (3*S*,6*S*)-6-dimethylamino-4,4-diphenyl-heptan-3-yl acetate (levacetylmethadol (LAAM)), (-)-6-dimethylamino-4,4-diphenol-3-heptanone (levomethadone), (-)-17-methyl-3-morphinanol (levorphanol), levophenacylmorphane, lofentanil, 5-(4-chlorophenyl)-2,5-dihydro-3*H*-imidazo[2,1-a]isoindol-5-ol (mazindol), *N*-(3-chloropropyl)-α-methylphenethyl-amine (mefenorex), meperidine, meptazinol, metazocine, methylmorphine, N,α-dimethylphenethylamine (metamphetamine), (±)-6-dimethylamino-4,4-diphenol-3-heptanone (methadone), methyl [2-phenyl-2-(2-piperidyl)acetate] (methylphenidate), 3,3-diethyl-5-methyl-2,4-piperidinedione (methyprylon), 2-(benz-hydrylsulfinyl)acetamide (modafinil), 4,5α-epoxy-17-methyl-7-morphinen-3,6α-diol (morphine), myrophine, (±)-*trans*-3-(1,1-dimethylheptyl)-7,8,10,10α-tetrahydro-1-hydroxy-6,6-dimethyl-6*H*-dibenzo-[*b*,*d*]pyran-9(6α*H*)-one (nabilone), nalbuphene, narceine, nicomorphine, norlevorphanol, 6-dimethyl-amino-4,4-diphenyl-3-hexanone (normethadone), normorphine, norpipanone, the exudation from plants belonging to the species *Papaver somniferum* (opium), 4,5α-epoxy-14-hydroxy-3-methoxy-17-methyl-6-morphinanone (oxycodone), oxymorphone, plants and parts of plants belonging to the species *Papaver somniferum* (including the subspecies *setigerum) (Papaver somniferum),* papaveretum, 2-imino-5-phenyl-4-oxazolidinone (pernoline), 1,2,3,4,5,6-hexahydro-6,11-dimethyl-3-(3-methyl-2-butenyl)-2,6-methano-3-benzazocin-8-ol (pentazocine), ethyl (1-methyl-4-phenyl-4-piperidinecarboxylate) (pethidine), phenadoxone, phenomorphane, phenazocine, phenoperidine, piminodine, pholcodeine, 3-methyl-2-phenylmorpholine (phenmetrazine), α,α-dimethyl-phenethylamine (phentermine), α-(2-piperidyl)-benzhydryl alcohol (pipradrol), 1'-(3-cyano-3,3-diphenylpropyl)[1,4'-bipiperidine]-4'-carboxamide (piritramide), profadol, proheptazine, promedol, properidine, propoxyphene, N-(1-methyl-2-piperidinoethyl)-N-(2-pyridyl)propionamide, methyl {3-[4-methoxycarbonyl-4-(*N*-phenylpropanamido)piperidino]-propanoate} (remifentanil), *N*-{4-methoxymethyl-1-[2-(2-thienyl)ethyl]-4-piperidyl}propionanilide (sufentanil), ethyl (2-dimethylamino-1-phenyl-3-cyclohexene-1-carboxylate) (tilidine, *cis* and *trans*)), tramadol, (1R*,2R*)-3-(3-dimethylamino-1-ethyl-2-methyl-propyl)-phenol, (1R,2R,4S)-2-(dimethylamino)-methyl-4-(p-fluorobenzyloxy)-1-(m-methoxyphenyl)cyclohexanol, in each case optionally in the form of corresponding stereoisomeric compounds and corresponding derivatives, in particular esters or ethers, and in each case physiologically acceptable compounds, in particular salts and solvates.

4. A dosage form according to claim 2 or claim 3, **characterised in that** the stimulant has been selected from the group consisting of amphetamine, norpseudoephedrine, methylphenidate and in each case optionally the corresponding physiologically acceptable compounds thereof, in particular the bases, salts and solvates thereof.

5. A dosage form according to any one of claims 2 to 4, **characterised in that** the antagonist used for an opiate or opioid is an antagonist selected from the group consisting of naloxone, naltrexone, nalmefene, nalide, nalmexone, nalorphine, naluphine and in each case corresponding physiologically acceptable compounds, in particular bases, salts and solvates.

6. A dosage form according to any one of claims 2 to 5, **characterised in that** the antagonist used for a stimulant is a neuroleptic, preferably selected from the group consisting of haloperidol, promethazine, fluphenazine, perphenazine, levomepromazine, thioridazine, perazine, chlorpromazine, chlorprothixine, zuclopentixol, flupentixol, prothipendyl, zotepine, benperidol, pipamperone, melperone and bromperidol.

7. A dosage form according to any one of claims 1 to 6, **characterised in that** it comprises at least one active ingredient at least partially in delayed-release form.

8. A dosage form according to any one of claims 1 to 7, **characterised in that** it comprises at least one coating resistant to gastric juices.

## Revendications

1. Forme d'administration solide, orale, protégée contre un usage abusif, comprenant au moins une substance active présentant un potentiel d'usage abusif et au moins un antagoniste de cette substance active, séparé dans l'espace de celle-ci, la ou les substances actives se trouvant dans au moins une sous-unité (a) et le ou les antagonistes se trouvant dans au moins une sous-unité (b) et le ou les antagonistes de la sous-unité (b) n'étant quasiment pas libérés lors d'une administration conforme à la destination de la forme d'administration dans le corps, **caractérisée en ce que**
(X) les deux sous-unités (a) et (b) se trouvent chacune sous forme multiparticulaire, les différentes formes multiparticulaires de la sous-unité (a) ou, selon le cas, de la sous-unité (b) étant façonnées de manière largement identique et ne pouvant être distinguées visuellement, sont le cas échéant transvasées dans des capsules ou mises en suspension dans un liquide ou un gel, ou
(Y) la sous-unité (a) forme un noyau qui est enrobé dans la sous-unité (b), cet enrobage présentant au moins un canal qui conduit du noyau à la surface de la forme d'administration, ou
(Z) la sous-unité (a) a la forme d'un comprimé dont l'âme et le cas échéant une des deux surfaces de base est revêtue par au moins une couche formant une barrière (b) contenant le/les antagoniste(s).

2. Forme d'administration selon la revendication 1, **caractérisée en ce que** la ou les substances actives sont des substances actives pharmaceutiques choisies dans le groupe constitué par les opiacés, les opioïdes, les stimulants et d'autres stupéfiants.

3. Forme d'administration selon la revendication 2, **caractérisée en ce que** la ou les substances actives sont choisies dans le groupe formé par le N-{1-[2-(4-éthyl-5-oxo-2-tétrazolin-1-yl)éthyl]-4-méthoxyméthyl-4-pipéridyl}propionanilide (alfentanil), l'allylprodine, l'alphaprodine, la 2-diéthylaminopropiophénone (amfépramone), la (±)-α-méthylphénéthylamine (amfétamine), le 2-(α-méthylphénéthylamino)-2-phénylacétonitrile (amfétaminil), l'aniléridine, l'apocodéine, la benzylmorphine, le bezitramide, le 17-cyclopropylméthyl-4,5α-époxy-7α[(S)-1-hydroxy-1,2,2-triméthylpropyl]-6-méthoxy-6,14-endoéthanomorphinan-3-ol (buprénorphine), le butorphanol, le (1S,2S)-2-amino-1-phényl-1-propanol (cathine/D-norpseudoéphédrine), le clonitazène, le (-)-méthyl-[3β-benzoyloxy-2β(1αH,5αH)-tropanecarboxylate] (cocaïne), le 4,5α-époxy-3-méthoxy-17-méthyl-7-morphinén-6α-ol (codéine), le cyclorphan, la cyprénorphine, la désomorphine, le dextromoramide, le propionate de (+)-(1-benzyl-3-diméthylamino-2-méthyl-1-phénylpropyl) (dextropropoxyphène), la dézocine, le diampromide, la diamorphine, le 4,5α-époxy-3-méthoxy-17-méthyl-6α-morphinanol (dihydrocodéine), le 4,5α-époxy-17-méthyl-3,6a-morphinanediol (dihydromorphine), le diménoxadol, le diméphétamol, le diméthylthiambutène, le butyrate de dioxaphétyle, la dipipanone, le (6aR,10aR)-6,6,9-triméthyl-3-pentyl-6a,7,8,10a-tétrahydro-6H-benzo[c]chromén-1-ol (dronabinol), l'eptazocine, l'éthoheptazine, l'éthylméthylthiambutène, le 4,5α-époxy-3-éthoxy-17-méthyl-7-morphinén-6α-ol (éthylmorphine), l'étonitazen, le 4,5α-époxy-7α-(1-hydroxy-1-méthylbutyl)-6-méthoxy-17-méthyl-6,14-endoéthénomorphinan-3-ol (étorphine), la N-éthyl-3-phényl-8,9,10-trinorbornan-2-ylamine (fencamfamine), la 7-[2-(α-méthylphénéthylamino)éthyl]-théophylline (fénétylline), le 3-(α-méthylphénéthylamino)propionitrile (fenproporex), le N-(1-phénéthyl-4-pipéridyl)propionanilide (fentanyl), l'héroïne, la 4,5α-époxy-3-méthoxy-17-méthyl-6-morphinanone (hydrocodone), la 4,5α-époxy-3-hydroxy-17-méthyl-6-morphinanone (hydromorphone), l'hydroxypéthidine, l'isométhadone, l'hydroxyméthylmorphinane, la 1-[4-(3-hydroxyphényl)-1-méthyl-4-pipéridyl]-1-propanone (cétobémidone), l'acétate de (3S,6S)-6-diméthylamino-4,4-diphénylheptan-3-yle (levacétylméthadol (LAAM)), la (-)-6-diméthylamino-4,4-diphényl-3-heptanone (lévométhadone), le (-)-17-méthyl-3-morphinanol (lévorphanol), le lévophénacylmorphane, le lofentanil, le 5-(4-chlorophényl)-2,5-dihydro-3H-imidazo[2,1-α]iso-indol-5-ol (mazindol), la N-(3-chloropropyl)-α-méthylphénéthylamine (méfénorex), la mépéridine, le meptazinol, la métazocine, la méthylmorphine, la N,α-diméthylphénéthylamine (métamfétamine), la (±)-6-diméthylamino-4,4-diphényl-3-heptanone (méthadone), le 2-phényl-2-(2-pipéridyl)acétate de méthyle (méthylphénidate), la 3,3-diéthyl-5-méthyl-2,4-pipéridinedione (méthyprylone), le 2-(benzhydrylsulfinyl)acétamide (modafinil), le 4,5α-époxy-17-méthyl-7-morphinène-3,6α-diol (morphine), la myrophine, la (±)-trans-3-(1,1-diméthylheptyl)-7,8,10,10α-tétrahydro-1-hydroxy-6,6-diméthyl-6H-dibenzo[b,d]pyrann-9(6αH)-one (nabilone), le nalbuphen, la narcéine, la nicomorphine, le norlévorphanol, la 6-diméthylamino-4,4-diphényl-3-hexanone (norméthadone), la normorphine, la norpipanone, le jus figé des plantes appartenant au type Papaver somniferum (opium), la 4,5α-époxy-14-hydroxy-3-méthoxy-17-méthyl-6-morphinanone (oxycodone), l'oxymorphone, les plantes et parties de plantes appartenant au type Papaver somniferum (y compris le sous-type setigerum) (Papaver somniferum), le papavérétum, la 2-imino-5-phényl-4-oxazolidinone (pernoline), le 1,2,3,4,5,6-hexahydro-6,11-diméthyl-3-(3-méthyl-2-butényl)-2,6-méthano-3-benzazocin-8-ol (pentazocine), le (1-méthyl-4-phényl-4-pipéridincarboxylate d'éthyle (péthidine), la phénadoxone, le phénomorphane, la phénazocine, la phénopéridine, la piminodine, la pholcodine, la 3-méthyl-2-phénylmorpholine (phenmétrazine), l'a,a-diméthylphénéthylamine (phentermine), l'acool α-(2-pipéridyl)benzhydrylique (pipradrol), le 1'- (3-cyano-3,3-diphénylpropyl)[1,4'-bipipéridine]-4'-carboxamide (piritramide), le profadol, la proheptazine, le promedol, la propéridine, lepropoxyphène, le N-(1-méthyl-2-pipéridinoéthyl)-N-(2-pyridyl)propionamide, le 3-[4-méthoxycarbonyl-4-(N-phénylpropanamido)pipéridino]propanoate de méthyle (Rémifentanil), le N-{4-méthoxyméthyl-1-[2-(2-thiényl)éthyl]-4-pipéridyl}propionanilide (sufentanil), le 2-diméthylamino-1-phényl-3-cyclohexène-1-carboxylate d'éthyle (tilidine (cis et trans)), le tramadol, le (1R*,2R*)-3-(3-diméthylamino-1-éthyl-2-méthylpropyl)-phénol, le (1R,2R,4S)-2-[diméthylamino)méthyl-4-(p-fluorobenzyloxy)-1-(m-méthoxyphényl)cyclohexanol, à chaque fois le cas échéant sous forme des composés stéréo-isomères correspondants ainsi que des dérivés correspondants, en particulier les esters ou les éthers et à chaque fois les composés physiologiquement acceptables, en particulier les sels et les solvants.

4. Forme d'administration selon la revendication 2 ou 3, **caractérisée en ce que** la substance stimulante a été choisie dans le groupe formé par l'amphétamine, la norpseudoéphédrine, le méthylphénidate et à chaque fois le cas échéant leurs composés physiologiquement acceptables correspondants, en particulier leurs bases, sels et solvates.

5. Forme d'administration selon l'une quelconque des revendications 2 à 4, **caractérisée en ce qu'**on utilise, comme antagoniste d'un opiacé ou d'un opioïde, un antagoniste choisi dans le groupe comprenant la naloxone, la naltrexone, le nalmefene, le nalide, le nalmexone, la nalorphine, la naluphine et à chaque fois des composés correspondants, physiologiquement acceptables, en particulier les bases, les sels ou les solvates.

6. Forme d'administration selon l'une quelconque des revendications 2 à 5, **caractérisée en ce qu'**on utilise, comme antagoniste, un stimulant, un neuroleptique, de préférence choisi dans le groupe constitué par l'haloperidol, la prométhazine, la fluphénazine, la perphénazine, la lévomépromazine, la thioridazine, la pérazine, la chlorpromazine, la chlorprothixène, le zuclopentixol, le flupentixol, le prothipendyle, la zotépine, le benpéridol, la pipampérone, la melpérone et le brompéridol.

7. Forme d'administration selon l'une quelconque des revendications 1 à 6, **caractérisée en ce qu'**elle présente au moins une substance active au moins partiellement dans une forme retard.

8. Forme d'administration selon l'une quelconque des revendications 1 à 7, **caractérisée en ce qu'**elle présente au moins un revêtement résistant au suc gastrique.
